# EUROPEAN PATENT SPECIFICATION

(11) **EP 1 349 572 B1**
(45) Date of publication and mention of the grant of the patent: **19.09.2007**
(21) Application number: 02729589.8
(22) Date of filing: 09.01.2002
(51) Int. Cl.: A61K 39/29

(54) **MANUFACTURING METHOD OF COMBINED VACCINE**
HERSTELLUNGSVERFAHREN FÜR KOMBINATIONSIMPFSTOFFE
METHODE DE FABRICATION D'UN VACCIN COMBINE

(30) Priority: 10.01.2001 KR 2001001290
(43) Date of publication of application: 08.10.2003
(73) Proprietor: LG Life Sciences Ltd., Seoul 150-721 (KR)
(72) Inventor: KIM, Kyu-wan, 305-340 Daejeon (KR); JI, Hyi-jeong, 305-340 Daejeon (KR); LEE, Youn-kyeong, 302-777 Daejeon (KR); KIM, Wan-kyu, 305-729 Daejeon (KR); LEE, Hee-ku, Yuseong-Gu, 305-751 Daejeon (KR); KIM, Won-kyum, 302-732 Daejeon (KR)
(74) Representative: Albrecht, Thomas
(86) International application number: PCT/KR2002/000034
(87) International publication number: WO 2002/055105

(56) References cited:
- EP-A- 0 339 667
- EP-A- 0 845 270
- WO-A-93/24148
- WO-A-98/00167
- WO-A-99/48525
- WO-A1-93/24148
- WO-A1-98/19702
- WO-A1-99/13906
- WO-A1-99/48525
- WO-A2-97/46255
- US-A- 4 578 270
- US-A- 4 649 192

## Description

### BACKGROUND OF THE INVENTION

### TECHNICAL FIELD

The present invention relates to manufacturing method of combined vaccine capable of preventing concurrently various diseases of infants such as diphtheria, tetanus, pertussis, hepatitis B, and other diseases, which should be prevented in infants.

### BACKGROUND ART

A combined vaccine is directed to a vaccine manufactured by combining protective antigens for each disease with respect to various other infection diseases or a vaccine manufactured by combining of various related antigens to prevent one infection disease.

As examples of the former, there are a DTP vaccine capable of enhancing an immunity with respect to diphtheria, tetanus and pertussis, a MMR vaccine capable of enhancing an immunity with respect to measles, mumps, and german measles, and so on. The above vaccines have been used for 20 years. As examples of the latter, there are a pneumococcal vaccine manufactured by combining 14 or 23 pneumococcal polysaccharides capable of enhancing an immunity to pneumonia, and a meningococcus vaccine manufactured by combining 4 meningococcal polysaccharides capable of enhancing an immunity to protect meningitis and so on. The above vaccines have been used for a few years.

The above vaccines have been used for many years since its development are well recognized with their high immunogenic effects and less side effect with respect to each infection disease and with their good adaptation to protect various diseases. Vaccine developers are newly developing various types of combined vaccines for the above reasons. As the above combined vaccines, there is a vaccine (International Publication No. WO 99/13906) manufactured by combining a DTP vaccine, bacterial encephalomeningitis vaccine (Hib vaccine), inactivated polio vaccine, and hepatitis B vaccine. A certain combined vaccine is developed by the combination of pneumococcal and meningococcal polysaccharides conjugated with protein. A combined vaccine capable of preventing from an intestinal infection with respect to each causing bacteria of cholera, typhoid, dysentery, and diarrhea will be developed soon.

The combined vaccine prepared in the way of combining each antigen for preventing various other infective diseases has advantages in that the number of vaccinations decreases, and the supply of vaccines is simple for thereby decreasing the cost. According to the infant vaccination schedule recommended by Pediatrics Association (1997), the infants gets many times of shot within 1 years after birth, and injection of vaccine is sometimes overlapped with different vaccines due to conveniance or illness. Therefore, it is important to inoculate the infants based on a proper method for thereby providing a certain convenience to both vaccinating persons and inoculated persons. In particular, as the standardized vaccination schedule, the DTP and the hepatitis B vaccinations are guided to perform three times of primary shots within the first year of infants and boost shots. In addition, since the vaccination schedules recommended are similar, the problem of overlaping shots can happen.

The inventors of the present invention performed a research for providing a certain convenience for vaccination to both the vaccinating persons and the inoculated persons by developing the combined vaccine of DTP and hepatitis B vaccines and stabilizing of the supply of the vaccine for thereby providing a vaccination benefit to more people.

Since the research on the combined vaccine is mostly directed to combining each component antigen from the vaccine products which is provided in the immunity and stability, the development of the combining method is important to minimize a variation in the reaction and immunity occuring due to an interaction between each protective antigen and adsorbent is important for the above research. With the completion of development, the homogeneity and stability of the combined vaccine formulations invented have been reviewed. In the present invention, the research has been performed based on the DTP vaccine and hepatitis B vaccine which are proved for their immunity and stability.

As a method for combining each component vaccine, there are a method (International Publication Nos. WO 99/13906 and WO 00/7623) of simply combining the products of each component vaccine, a method (International Publication No. WO 99/13906) of administrating simultaneously when the vaccination is performed, using the specially designed container, and a method of manufacturing the combined vaccine by mixing each component of vaccine and ingredients in one formulation. Concerning the vaccine supply, the usage of the combined vaccine prepared by the former two methods are similar to the use of each monovalent vaccine singularly it needs more attention to handle vaccines than each monovalent vaccine, so that there is not an advantage in using this type of the combined vaccine. In addition, it is impossible to perform a research with respect to the variation of the immunity and the occuring of the side effects. Therefore, it is preferable that each vaccine component is mixed in one formulation as one product on the research of the combined vaccine.

The purpose of the present invention is to provide a manufacturing method of combined vaccine capable of preventing various diseases of infants including diphtheria, tetanus, pertussis and hepatitis B, which should be prevent in infants.

### DISCLOSURE OF THE INVENTION

Accordingly, it is an object of the present invention to provide manufacturing method of combined vaccine for concurrently preventing various diseases of infants such as diphtheria, tetanus, pertussis and hepatitis B, which should be prevent in infants.

In order to achieve the above object, there is provided a manufacturing method of a combined vaccine, as defined in the appendant claims.

### BRIEF DESCRIPTION OF THE DRAWINGS

The present invention will become better understood with reference to the accompanying drawings which are given only by way of illustration and thus does not limit to the present invention, wherein;

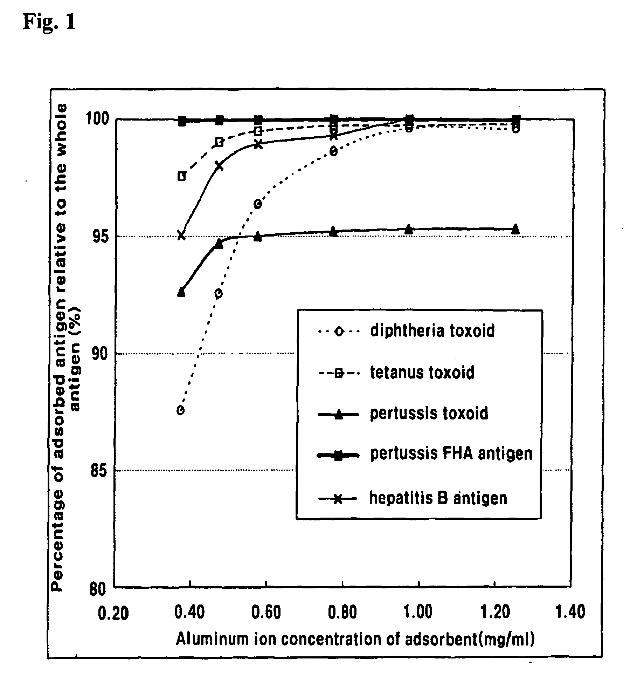

Fig. 1 is a view illustrating an adsorption ratio of each component antigen based on concentration of an adsorbent. Diphtheria toxoid, tetanus toxoid, and hepatitis B virus surface antigen are a components of diphtheria antigen, tetanus antigen and hepatitis B antigen, and pertussis toxoid and pertussis FHA antigen(pertussis thready shape blood agglutinin corpuscle) are each component of a purified pertussis antigen;

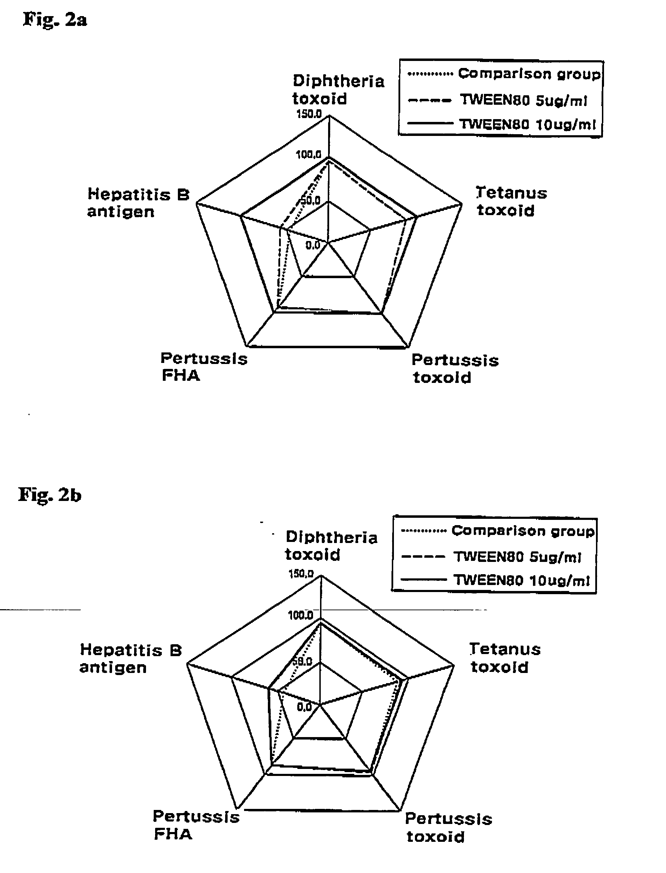

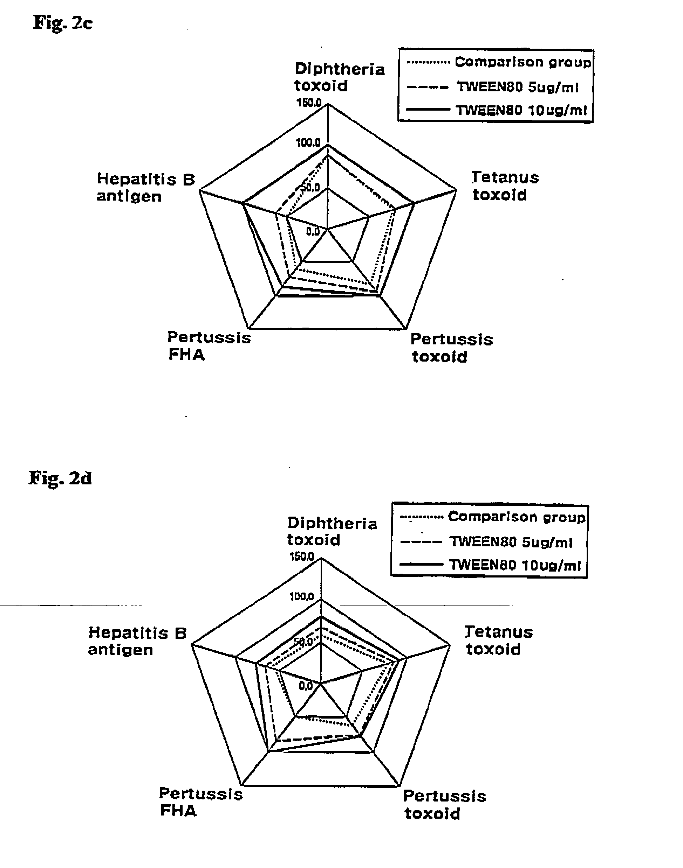

Figs. 2a to 2d are views illustrating an antigenicity and immunity based on an adsorption method wherein sample 1 is a sample in which surplus aluminum hydroxide gel is added after a combination of each component vaccine is completed, and sample 2 is a sample in which the said adsorbent of the same concentration is previously added before the combination is completed; Figs 2a and 2b are views illustrating the relative antigenicity of samples 1 and 2 respectively and, Figs 2c and 2d are views illustrating the relative level of antibody formation against each antigen of samples 1 and 2, respectively.

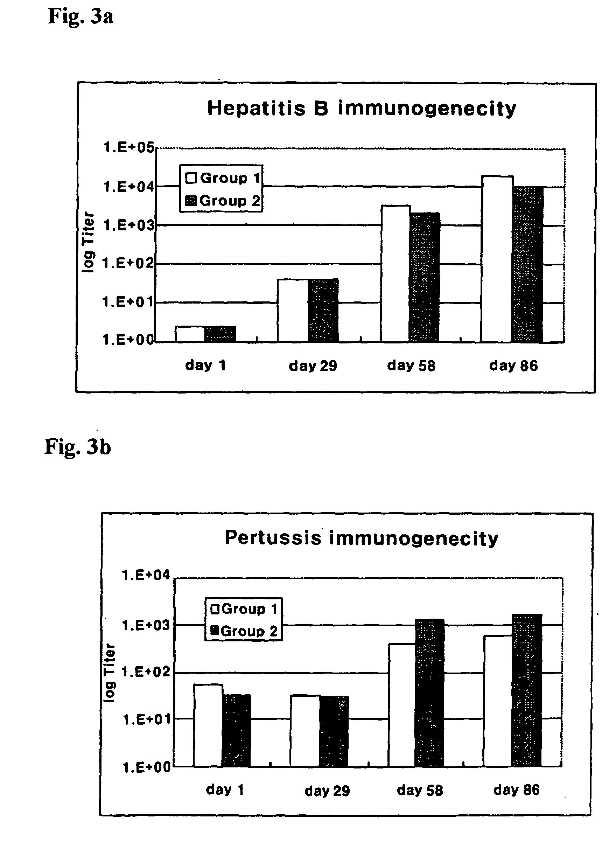

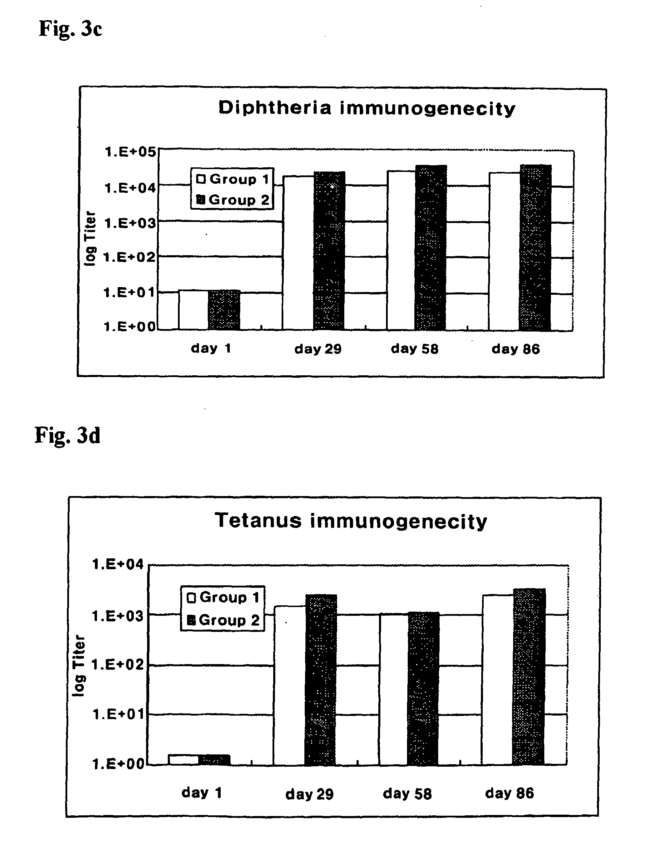

Fig. 3 is a view illustrating the level of antibody formation against each antigen in the serum obtained from monkeys administered a combined vaccine prepared according to the present invention or conccurently each vaccine product of DTP and hepatitis B based on the date of collecting blood samples from each monkey. Group 1 is designated that each vaccine of DTP and hepatitis B are concurrently administered, and group 2 is designated that a combined vaccine is administered.

### MODES FOR CARRYING OUT THE PREFERRED EMBODIMENTS

As the method for manufacturing the combined vaccine containing each vaccine component as one product, the above method is divided into two steps: a method for the step of combining the antigens which form each component vaccine and performing an adsorption using an adsorbent and a method for the step of combining the previously adsorbed antigens, in the case that an adsorbent is used as a component in the said method. However, in the case that each component antigen are first mixed, an adsorption ability with respect to the adsorbent of each antigen may decrease due to the interaction among antigens and ingredients in solution. In addition, the adsorption between the adsorbent and component antigen is optimized under each independent condition. If the above adsorption processes are concurrently performed, the adsorption ratio may be decreased due to a difference in the adsorption condition of each component antigen. Since the interrelationship between the adsorption ratio with respect to the adsorbent of the component antigen and the immunity of the vaccine is in inverse proportion, the immunity of the combined vaccine, which is manufactured in the process performed concurrently, may be decreased compared to the immunity of each monovalent vaccine.

Therefore, the inventors of the present invention have judged that it is proper to manufacture a combined vaccine by preparing a bulk solution of each component antigen, independently performing an adsorption of each antigen and combining the adsorbed antigens together.

In addition, in the case that each component antigen is combined in each adsorption type, the final concentration of each component antigen in the combined vaccine should be the same as the concentration of the component antigen in each conventional monovalent vaccine, to compare the immunity and the characteristics of vaccine antigens in vaccine products. Therefore, to manufacture the combined vaccine, the component antigen more concentrated than a single vaccine is adsorbed and then combined. The DTP vaccine is manufactured based on an adsorption using the antigen which is concentrated 1.5~2 times, and the hepatitis B vaccine is manufactured using the antigen which is concentrated 2~3 times. The final concentration of each component antigen of the combined vaccine according to the present invention is coincided with the concentration of each single vaccine adjusting the combining ratio of each antigen.

According to the present invention, the inventors performed the various studies of combinations for developing the combined vaccine. In this case, the final content of each immune component in a vaccine product is the same as the content of the monovalent vaccine. In addition, the change of the adsorption ratio of each antigen is monitored by controlling the contents of the adsorbent and other components. Each sample which has a less change in the adsorption ratio is immunized into small animals to compare the level of the antibody formation. A proper combining method was selected by reviewing the result of antibody formation in small animals.

In addition, as a result of the review with respect to the adsorbents of the currently commercial DTP vaccine and the hepatitis B vaccine, aluminum hydroxide gel; and aluminum phosphate gel were mainly used. It is known that the adsorption of the protein to each aluminum gel is caused by a surface electric charge of each protein and a gel component. And it is also known that the aluminum hydroxide gel has a positive surface electric charge in a physiological pH range, and aluminum phosphate gel has a negative surface electric charge ("Vaccine Design - The subunit and adjuvant approach", ed. By M.F. Powell & M.J. Newman, 1995, p229-239). In the case that the above two types of aluminum gels are concurrently used, a certain interaction occurs, so that the size of particle in the solution may be increased, and the adsorption capacity to protein may be decreased. In addition, the surface electric charge of each component antigen of the hepatitis B vaccine and combined vaccine has generally a negative surface electric charge in a physiological pH range. Therefore, the aluminum hydroxide gel is proper as an adsorbent.

In the conventional art, the component of the adsorbent such as the aluminum hydroxide gel and aluminum phosphate gel was co-used (International Publication No. WO 93/24148) but based on the above reason, the inventors of the present invention judged that the adsorbent of the combined vaccine should have the same salt type and the aluminum hydroxide gel which is used as an adsorbent is an important factor for obtaining a certain adsorption ratio of each component antigen.

In addition, in the combined vaccine prepared by the present invention, other component antigens except hepatitis B antigen include proteins as a main component, while, in the case of the hepatitis B vaccine, the antigen exists generally in a particle type which includes a phospholipid layer. Therefore, it is preferred that when manufacturing the hepatitis B vaccine for a combined vaccine prepared by the present invention, in order to decrease a certain interference by other antigens with respect to the phospholipid component of the hepatitis B vaccine antigen which includes a phospholipid layer, a neutral surfactant such as Polysorbate 20 (Tween 20), Polysorbate 80 (Tween 80) and Triton X-100 are added.

Since the titer of the hepatitis B vaccine tends to decrease in the case that the amount of the neutral surfactant is high, it is preferred that the neutral surfactant is added at the mass ratio below 50% with respect to protein amount of the hepatitis B surface antigen but the above amount is not limited thereto.

In the present invention, Corynebacterium diphtheria, PW No. 8 as an antigen of the diphtheria is prepared and cultivated in a proper culture medium. The diphtheria toxoid is preferred, which is obtained by detoxifying the diphtheria toxin which is purified by a conventional method. The amount of the antigen is preferably 10~25 Lf based on the pediatric dose. As a tetanus antigen, Clostridium tetanii, Harvard, is cultivated in a proper culture medium under anaerobic condition. The tetanus toxoid which is obtained by detoxifying the tetanus toxin purified by the conventional method is proper. The amount of the antigen is preferably 1~5Lf based on the pediatric dose. In addition, the Pertussis antigen is cultivated in a proper culture medium using Bordetella pertussis, Tohama phase I, and multiple kinds of antigen protein including a Pertussis toxoid are purified by a conventional method in a culture supernatent and detoxified. Purified Pertussis antigens or a whole cell Pertussis antigen is proper. Here, the manufacturing method of a combined vaccine is provided in which in the case of the multiple kinds of antigens, the total amount of the antigens is below 20µgPN based on the pediatric dose, and in the case of the whole cell Pertussis antigen, the amount of the antigen is preferably below 20 OE based on the pediatric dose. The present invention is further directed to a method for manufacturing a combined vaccine in which purified multiple antigen proteins include a detoxified Pertussis toxoid and filamentous hemagglutinin (FHA) antigen.

Preferably, the present invention is directed to a method for manufacturing a combined vaccine in such a manner that the amount of recombinant hepatitis B virus surface antigen, which manufactured by a genetic engineering method as a hepatitis B antigen, is 5~10ug based on the pediatric dose. More preferably, the present invention is directed to a method for manufacturing in such a manner that the hepatitis B surface antigen is adsorbed to an adsorbent by mixing with stirring at 2~8°C for 3~20 hours. In order to find the optimum content of adsorbant for manufacturing of the combined vaccine, the adsorption ratio for each antigen component was analyzed at each concentration of adsorbant. As a result, when the final aluminum ion concentration is below 0.5mg/ml at the time when the final combination is completed, the adsorption ratio of each antigen was relatively decreased (as shown in Fig. 1). In addition, the aluminum ion concentration is stipulated to be under 1.25mg/ml in the aluminum gel used for the vaccine by regulation (WHO, "Requirements for diphtheria, tetanus, pertussis and combined vaccines" in Technical Report Series No. 800, 1990, p87~179). Therefore, as the aluminum hydroxide gel, the concentration of the final aluminum ion is preferably in the range of 0.5~1.25mg/ml and more preferably in the range of 0.7mg/ml.

In addition, in order to prevent a possibility that the absorption ratio of each component antigen is decreased by a repulsive force caused among each component antigen, even after the combination of each component is completed, the antigenicity and the level of antibody formation of each component antigen are converted to the relative percentage to compare those values between two samples: a sample that the other component except an aluminum hydroxide gel is previously combined and then suplus aluminum hydroxide gel is additionally added and a sample that the adsorbent of the same concentration is previously added before the combination is completed. The change of the antigenicity and the level of antibody formation decreases when the surplus adsorbant is added even after the combination of each component is completed (as shown in Fig. 2).

In addition, in the case of the sample in which the polysorbate 80 (tween 80) is added, the antigenicity and titer with respect to the hepatitis B were maintained (as shown in Fig. 2). It is expected that a similar result may be obtained even when neutral surfactants such as polysorbate 20 and triton X-100 are used whithin proper concentration. As shown in Fig. 2, the sample 1 and sample 2 are prepared in the way of that surplus aluminum hydroxide gel is additionally added after the combination of each component is completed, and that the adsorbent of the same concentration is previously added before the combination is completed-, respectively.

Preferably, the present invention is directed to a method for manufacturing a combined vaccine in which the aluminum ion concentration of the aluminum hydroxide gel is in the range of 0.5~1.25mg/ml. More preferably, the present invention is directed to a method for manufacturing a combined vaccine which includes a step of adding surplus adsorbent in the range that does not exceed the concentration range of the aluminum ion of 0.5~1.25 mg/ml after protective antigens adsorbed to the adsorbent are combined. In a preferred embodiment of the present invention, it is possible to maintain an inherent adsorption of each component antigen in the combined vaccine and to increase an immunity.

As the Pertussis antigen, a purified Pertussis antigen is generally used in Korea and several other countries. Since some American countries and the third world countries use the whole cell Pertussis antigen which is detoxified. Therefore, in the present invention, the applicable range of the combined vaccine is widened by the study on the combining method using whole cell pertussis antigen. In this case, the combining was performed in the same method as using the mentioned purified Pertussis antigen for thereby obtaining a sample capable of maintaining an immunity of each component(as shown in Table 1).

In the case of the DTP vaccine which includes a whole cell Pertussis antigen, a aluminum phosphate gel is generally used as an adsorbent. However, in order to manufacture a combined vaccine with respect to the hepatitis B antigen, the DTP vaccine was manufactured using the aluminum hydroxide gel. In this case, the immunity with respect to the Pertussis was decreased, but when a surplus aluminum hydroxide gel was additionally added after the combination of each component vaccine was completed (sample A), it was possible to maintain a desired immunity. As a reference, the sample B is prepared in the way of that the adsorbent of the same concentration was previously added before the combination was completed.

The safety and efficacy of the combined vaccine manufactured according to the present invention was proved based on the following methods. First, overdosage of combined vaccine was administrated to a rodent, and it was checked that a lesion was not observed as a result of the biopsy (The result of the same is not provided). In addition, the antibody level against each antigen was measured in a group (group 2) in which a combined vaccine was administrated to a monkey and in a group (group 1) in which each vaccine of DTP and hepatitis B was concurrently administrated. As a result of the t-test with respect to the antibody amount between two groups, when comparing the result of the group 2 with the result of the group 1, it was evaluated that the combined vaccine showed the equal or better immunogenecity against each antigen to concurrently injection group of each vaccine (as shown in Fig. 3. The result of the statistic is not provided).

In conclusion, the combining method was developed in the present invention, which an interaction among different immune components was minimized, and each immunogenic effect did not decrease. When dividing each immune component used for the combined vaccine based on physical and chemical properties, the immunogenic components may be divided into a protein antigen (diphtheria antigen, tetanus antigen, purified Pertussis antigen), an antigen composed of protein and phospholipid layer (hepatitis B antigen), and an antigen composed of killed whole cell (a whole cell Pertussis antigen), etc. Therefore, it may be predicted that a combination with other antigen of single vaccine may be implemented based on the physical and chemical characteristics of components. A combination of an additional vaccine may be possible with respect to the diseases (hemophilus influenza, polio), which should be prevented in the infants by the combined vaccines prepared according to the present invention.

The body vaccination period of the combined vaccine agent according to the present invention may be the previous vaccination periods of the DTP vaccine or the hepatitis B vaccine. In the case that there is the hepatitis B antigen in the mother's body, it is preferred to inoculate three times of second, fourth and sixth months. In the case that there is not the hepatitis B in the mother's body, as a proper vaccination period, the hepatitis B vaccine is singularly inoculated after birth, and then the additional vaccination is performed using a combined vaccine. A proper vaccination with respect to the infants can be performed by a muscle or hypodermic injection method. The doze of the antigen of diphtheria, tetanus, Pertussis and hepatitis B respectively in the combined vaccine according to the present invention is the same as the doze for the infant of the antigen of each single vaccine.

### Example 1

### Manufacture of hepatitis B antigen

The yeast cell which is capable of expressing the hepatitis B surface antigen by genetic engineering method is intensively cultivated. 1kg of the precipitate of yeast cells which is obtained by the centrifugation is diluted at a ratio of 1:2 in a buffer solution (0.5M NaCl, 10mM EDTA, 0.01% Thimerosal, 0.1M Phosphate, pH 7.0). The diluted solution flows through a glass bead beator (or Dynomil) for thereby breaking cell walls and so on. The obtained solution is added with a neutral surfactant (Tween group or Triton group) by 0.5% and is uniformly mixed by stirring at 4°C. Sodium hydroxide was added to the resultant solution for thereby obtaining pH 11 and is then mixed by stirring at 4°C for 5 hours. A diluted hydrochloric acid was added to the resultant solution for thereby obtaining pH 4. The precipitate was removed by a centrifugation at 6000rpm for 15 minutes using the centrifugal machine (ROTOR: JA-14, Beckman Inc. USA), and the upper solution including the hepatitis B surface antigen was obtained. The pH of the upper solution was made at 7, and then silica was added thereto and was mixed at 4~25°C for 3-16 hours, so that the hepatitis B surface antigen was adsorbed to the silica. The silica gel which is preferably used in the present invention is Aerosil 380(Degussa, USA) which includes fine hydration silica or anhydrous silica having a valid surface area of 100~500mm²/g. In order to remove contaminants from the silica to which the hepatitis B surface antigen is adsorbed, the resultant solution was washed two times using sodium phosphate - sodium chloride buffer solution of pH 7. The washed silica was contacted in the sodium carbonate buffer solution of pH 9.6 for about 2 hours for thereby desorption of a surface antigen. The thus separated surface antigen had a protein purity of above 90% in the solution. The resultant solution was flown in the DEAE-Sepharose (PHAMACIA, Sweden) which was balanced using the said sodium buffer solution for thereby specifically attaching a surface antigen and removing the substances separated in the column using the said buffer solution. Contaminants slightly attached on the column was eluted using the buffer solution including sodium chloride of 0.05~0.1M. Thereafter, the hepatitis B surface antigen was eluted using the said buffer solution including the sodium chloride of 0.2M. The thus eluted solution was concentrated using a ultrafiltration membrane which is capable of separating the substance of molecular weight above than 100,000, and the precipitate was separated by centrifugation and removed, and the upper solution was collected, and the gel permeation chromatography (Sepharose CL-4B, PHAMACIA, Sweden) was performed with respect to the collected upper solution. The fractions which included a pure surface antigen were confirmed by electrophorsis and were used as the hepatitis B antigen. In addition, in order to decrease an interaction with each of the hepatitis surface antigen, the tween 80 was added to 0, 5, 10µg per 1ml, and then the effect of tween 80 was observed (as shown in Fig. 2).

### Example 2

### Manufacture of diphtheria toxoid

The corynebacterium diphtheria PW No. 8 was cultured at 35°C for 24 hours in the nutrition agar culture medium (DIFCO, USA) and was subcultured two times. One of the colony was cultured at 35°C for 24 hours in 2ml of the brain heart infusion culture medium (DIFCO, USA), and 1.2ml of it was inoculated to the modified Muller culture medium (refer to: Stainer, et. al, Canadian J. Microbiol., 14:155, 1968) of 300ml and was cultured at 35°C for 36 hours. After the culture, cells were removed from the culturing solution, and the toxin solution was collected and it was added with ammonium sulfate at 4°C. The final concentration was made in 25(w/v)%, and pH was made in 8.0. The culturing solution having adjusted pH and concentration of salt was dropped into the phenyl-sepharose column which was previously balanced using 10mM tris buffer solution pH 8.0 including 25(w/v)% ammonium sulfate. The buffer solution same as the column balance solution and 10mM tris buffer solution(pH 8.0) including 15(w/v)% ammonium sulfate were sequentially flown to the column for thereby removing impurities. The toxin was eluted using 10mM tris buffer solution(pH 8.0) which did not include salt. The eluted diphtheria toxin solution was collected and was dialyzed to 10mM phosphate buffer solution(pH 7.4) including sodium chloride of 150mM. After the dialyzing process was completed, formalin was added, so that the final concentration was 0.05(w/v)%, and the resultant solution was reacted at 37°C for 1 hour, and lysine was added for thereby obtaining the final concentration of 0.05M and then, the resultant solution was detoxified at 37°C for 4 weeks. The toxoid solution was dialyzed into 10mM phosphate buffer solution(pH 7.4) with sodium chloride for thereby fully removing the formalin, and thimerosal was added to have 0.01(w/v)% of the final concentration. The resultant solution was used as diphtheria toxoid solution.

### Example 3

### Manufacture of tetanus toxoid

The *Clostridium tetanii* (Harvard strain) was cultured by molten agar method with sterilized liver-bile agar medium (DIFCO, USA). A few colonies from above culture were inoculated into brain heart infusion culture medium (DIFCO, USA) including 0.3(w/v)% yeast extraction (DIFCO, USA) of 2ml having decreased oxygen level and were cultured at 35°C for 24 hours in anaerobic state. And then, the culture solution was inoculated into fully nitrogen-satured brain heart infusion culture medium (DIFCO, USA) 500ml including 0.3(w/v)% yeast extraction (DIFCO, USA) and were cultured at 35°C for 7 days in anaerobic state. After the culture, cells were removed from the culture solution, the toxin solution was collected and added with ammonium sulfate at 4°C for thereby obtaining the final concentration of 60(w/v)% and was mixed for over 24 hours and was fully mixed for thereby obtaining a precipitate by centrifugation. The precipitate was dissolved in a small amount of distilled water, and the insoluble material was removed. The resultant solution was dropped into the sephagrill S-100 column which was previously balanced using 10mM tris buffer solution(pH 8.0) including 0.5M sodium chloride. The same buffer solution was flown for thereby eluting the separated toxin. The tetanus toxin solution was collected and dialyzed into 10mM phosphate buffer solution(pH 7.4) including 150mM sodium chloride. After the dialysis, formalin was added to have the final concentration of 0.025(w/v)% and was reacted at 37°C for 1 hour. Thereafter, lysine and sodium hydrogencarbonate were added to have the final concentration of 0.05mM and 0.04M, respectively, and was matured at 37°C for 4 weeks and was detoxified. After the detoxification, the toxoid solution was dialyzed into 10mM phosphate buffer solution (pH 7.4) including 150mM sodium chloride, and formalin was fully removed. Thimerosal was added to have the final concentration of 0.01(w/v)% and was used as tetanus toxoid solution.

### Example 4

### Manufacture of purified Pertussis antigen protein

*Bordetella pertussis, Tohama* phase I was cultured in Bodet-Gengo agar culture medium(DIFCO, USA) including 15% rabbit blood at 35°C for 72 hours and was passaged two times. A few colonies were inoculated into 50ml of stanor-sholte culture medium and was cultured at 35°C for 48 hours. The thusly cultured solution were re-inoculated in 500ml of changed stanor-sholte culture medium(refer to Imazumi et al., INFECT.-IMMUN., 1983, vol 41, pp. 1138) and was cultured at 35°C for 36 hours. 10% thimerosal aqueous solution was added to the cultured solution to have the final concentration of 0.01 % for thereby preventing the growth of the Pertussis bacteria, and then the cells were removed by centrifugation. Three times volume of distilled water was added to the resultant solution, and was mixed well. The current pH was decreased to pH 6.0 using 1N sulfuric acid. The resultant solution was dropped into CM-sepharose column which was previously balanced using 10mM sodium phosphate buffer of pH 6.0. The buffer solution same as the column balance solution was flown to the column for thereby removing the substances which were not coupled to the column, and the impurities which were slightly coupled to the column were removed using the buffer solution same as the column balance solution including 100mM sodium chloride. When the impurities were not eluted anymore through the column, the bound materials were eluted with linear gradient formed by the column balance solution including 100mM sodium chloride and 600mM sodium chloride which have the same volume, so that a fraction including Pertussis antigen protein such as Pertussis toxin and FHA (filamentous hemagglutinin) was separated. The antigen fraction was diluted using 10mM sodium phosphate buffer solution(pH 8.0) of two times volume to have pH 8.0. The resultant solution was dropped into hydroxy apatite column which was previously balanced using 10mM sodium phosphate buffer solution of pH 8.0. The buffer solution same as the column balance solution including 100mM sodium chloride was flown to the column for thereby removing the substances which were not coupled to the column. 80mM sodium phosphate buffer solution of pH 8.0 including 100mM sodium chloride was flown at the same rate as the dropping rate for thereby separating Pertussis toxin fraction. When the Pertussis toxin was separated, 250mM sodium phosphate buffer solution of pH 8.0 including 100mM sodium chloride was flown at the same rate as the dropping rate for thereby separating FHA(filamentous hemagglutinin) fraction. Separated Pertussis toxin and FHA(filamentous hemagglutinin) were dialyzed in 10mM sodium phosphate buffer solution(pH 7.4) including 0.15% sodium chloride at 4°C. Glycerol and glutal aldehyde were added to dialyzed Pertussis toxin sample solution to have the final concentration of 50(w/v)% and 0.05(w/v)% and were detoxified at 37°C for 4 hours. Sodium aspartate was added to have the final concentration of 0.025M for thereby completing the detoxifying process. Glycerol and formalin were added into the dialyzed FHA (filamentous hemagglutinin) sample solution to have 50(w/v)% and 0.025(w/v)% and were detoxified at 37°C for 24 hours. Lysine was added to have the final concentration of 0.025M, so that the detoxifying process was completed. Each sample solution was dialyzed into 10mM sodium phosphate buffer solution (pH 7.4) including 0.15% sodium chloride at room temperature. Thimerosal was added to have the final concentration of 0.01(w/v)%. Thereafter, detoxified Pertussis toxin sample solution and FHA (filamentous hemagglutinin) sample solution were mixed at a ratio of 1:4 and were used as a Pertussis antigen protein.

### Example 5

### Manufacture of a whole cell Pertussis antigen

Pertussis bacteria, *Bordetella pertussis, Tohama* phase I was cultured in Bodet-Gengo agar culture medium(DIFCO, USA) including 15% rabbit blood at 35°C for 72 hours and was passaged two times. Some of the colonies were inoculated into 50ml of Stanor-sholte culture medium and were cultured at 35°C for 48 hours. The resultant solution was re-inoculated in the changed stanor-sholte culture medium(refer to Imazumi et al., INFECT.-IMMUN., 1983, vol 41, pp. 1138) of 500ml and were cultured at 35°C for 36 hours. 10% thimerosal aqueous solution was added into the culture solution to have the final concentration of 0.01% for thereby preventing the growth of the Pertussis bacteria and collecting somatic based on the centrifugation. The collected somatic solution was dialyzed in 10mM sodium phosphate buffer solution(pH 7.4) including 0.15% sodium chloride at 4°C. Formalin was added into the dialyzed Pertussis somatic solution to have the final concentration of 0.025(w/v)% and was detoxified at 37°C for 4 weeks. Lysine was added to have the final concentration of 0.025M for thereby completing the detoxifying process. The sample solution was dialyzed into 10mM sodium phosphate buffer solution(pH 7.4) including sodium chloride of 0.15%, and thimerosal was added to have the final concentration of 0.01(w/v)% and was used as the whole cell Pertussis antigen.

### Example 6

### Manufacture of combined vaccine including purified Pertussis antigen

### Step 1. Manufacture of hepatitis B vaccine

The hepatitis B vaccine was manufactured using the hepatitis B surface antigen manufactured in Example 1. aluminum hydroxide solution was manufactured by adding aluminum hydroxide gel to the phosphate buffer solution. The resultant solution was slowly mixed, and the above antigen solution was dropped and was mixed by stirring slowly at 4°C for 15 hours for thereby manufacturing hepatitis B vaccine. At this time, the amount of the hepatitis surface antigen was 60µg/ml which was high concentrated three times compared to the amount of the common hepatitis B surface antigen. In the case of the first sample, the amount of the aluminum ion in the aluminum hydroxide gel was 0.9mg/ml. In the case of the second sample, the amount of the aluminum ion in the aluminum hydroxide gel was 1.5mg/ml(as shown in Fig. 2).

### Step 2. Manufacture of DTP combined vaccine

The DTP combined vaccine was manufactured using each antigen manufactured in the second, third and fourth examples. The aluminum hydroxide solution was manufactured by adding aluminum hydroxide gel to the phosphate buffer solution in the conventional method. As the resultant solution was slowly mixed, each antigen solution was dropped into each aluminium hydroxide solution and was mixed by stirring slowly for thereby implementing a uniform adsorption, so that the DTP combined vaccine was manufactured. At this time, the amount of each antigen was concentrated 1.5 times high compared to the amount of each component antigen of common DTP vaccine. The amount of the aluminum ion in the aluminum hydroxide gel was 0.3mg/ml.

### Step 3. Manufacture of DTP-hepatitis B combined vaccine

Each DTP vaccine and hepatitis B vaccine manufactured in Steps 1 and 2 were slowly mixed at the volume ratio of 2:1. In the case of the first sample, surplus aluminum hydroxide gel was added so that the amount of the aluminum ion in the aluminum hydroxide gel was finally 0.7mg/ml, and was continuously mixed by stirring at 25°C for 1 hour for thereby manufacturing a stable combined vaccine which does not interact with other components. In the case of the second sample, surplus aluminum hydroxide gel was not added and was continuously mixed by stirring at 25°C for 1 hour for thereby manufacturing combined vaccine. The amounts of the aluminum ions in the aluminum hydroxide gel of the combined vaccines of the first and second samples were 0.7mg/ml. For the test of antibody titer, the composition of the combined vaccine was 20µg of the hepatitis B surface antigen, 25Lf of diphtheria toxoid, 3Lf of tetanus toxoid, 2.5µgPN of Pertussis toxoid, and 10µgPN of Pertussis FHA antigen(Pertussis FHA(filamentous hemagglutinin) antigen based on 1 ml(as shown In Fig. 2).

### Example 7

### Manufacture of combined vaccine including a whole cell Pertussis antigen

### Step 1. Manufacture of hepatitis B vaccine

The hepatitis B vaccine was manufactured using the hepatitis B surface antigen manufactured in Example 1. An aluminum hydroxide gel was added to phosphate buffer solution for thereby manufacturing the aluminum hydroxide solution. As the solution was slowly mixed, the above antigen solution was dropped and was fully mixed at 4°C for 15 hours for thereby manufacturing the hepatitis B vaccine. At this time, the amount of the hepatitis B surface antigen was 60µg/ml which was concentrated three times high compared to the amount of the common hepatitis B vaccine. In the case of the first sample, the amount of the aluminum ion was 0.9mg/ml in the aluminum hydroxide gel. In the case of the second sample, the amount of the aluminum ion was 1.5mg/ml in the aluminum hydroxide gel(as shown in Fig. 2).

### Step 2. Manufacture of DTP combined vaccine

The DTP combined vaccine was manufactured using each antigen manufactured in the Examples 2, 3 and 5. The aluminum hydroxide gel was added to the phosphate buffer solution in the conventional method for thereby manufacturing an aluminum hydroxide solution. As the resultant solution was slowly mixed, the above each antigen solution was dropped into each aluminium hydroxide solution and was fully mixed. A uniform adsorption was implemented for thereby manufacturing a DTP combined vaccine. At this time, the amount of each antigen was concentrated 1.5 times high compared to the amount of each component antigen of the common DTP vaccine. The amount of the aluminum ion was 0.3mg/ml in the aluminum hydroxide gel.

### Step 3. Manufacture of DTP-hepatitis B combined vaccine

Each DTP vaccine and hepatitis B vaccine manufactured in Steps 1 and 2 were slowly mixed at a volume ratio of 2:1. In the case of the first sample, a surplus aluminum hydroxide gel was added so that the amount of the aluminum ion was finally 0.7mg/ml in the aluminum hydroxide gel, and the resultant solution was continuously mixed at 25°C for 1 hour for thereby manufacturing a stable combined vaccine which does not interact with other components. In the case of the second sample, surplus aluminum hydroxide gel was not added, and the solution was continuously mixed at 25°C for 1 hour for thereby manufacturing a combined vaccine. The amounts of the aluminum ions in the aluminum hydroxide gel of the combined vaccines of the samples 1 and 2 were 0.7mg/ml. For the test of antibody titer, the composition of the combined vaccine was 20µg of the hepatitis B surface antigen, 50Lf of diphtheria toxoid, 10Lf of tetanus toxoid, and 200E of a whole cell Pertussis antigen based on 1ml(as shown in Fig. 2).

### Example 8. Test of antigenicity in combined vaccine

The test of antigenicity in combined vaccine manufactured by the examples 6 and 7 was performed by the Enzyme Immuno Assay (EIA) using monoclonal antibody and polyclonal antibody by comparison to the standard of each antigen with respect to each antigen content.

In addition, the antigen adsorbed to the aluminum salt was removed by the centrifugation for measuring the adsorption ratio of each antigen, and the amount of the separated antigen was measured using the upper solution.

The average of each result were shown in Figs. 1 and 2 shows relative antigenicity of each antigen.

### Example 9. Test of antibody formation by combined vaccine

### Test of antibody formation by hepatitis B surface antigen

The combined vaccine and hepatitis B vaccine manufactured in Examples 6 and 7 were inoculated to ICR mice in which 16 mice were grouped as the group 1, and a blood was collected from the ICR mice after 18 days. Serum was separated from the blood. The antibody level against the hepatitis B surface antigen was obtained based on the unit of IU/ml using the EIA(Enzyme Immuno Assay) and calculated as in geomean antibody titer. The titer of the combined vaccine was computed with respect to the hepatitis B vaccine.

Fig. 2c and 2d shows relative antibody titer of combined vaccine with repect to each antigen and Table 1 shows the values of each titer.

### Test of antibody formation by diphtheria antigen

The combined vaccine and DTP vaccine manufactured in Examples 6 and 7 were inoculated to the ICR mice in which 16 mice are grouped as the group 1. The blood was collected from the CIR mice after 28 days. Serum was separated from the blood. The antibody level against the diphtheria antigen of the serum was measured using the EIA(Enzyme Immuno Assay) based on the unit of IU/ml and calculated as in geomean antibody titer. Thereafter, the titer of the combined vaccine was measured compared to the DTP vaccine.

Fig. 2c and 2d shows relative antibody titer of combined vaccine with repect to each antige, and Table 1 shows the values of each titer.

### Test of antibody formation by tetanus antigen

The combined vaccine and DTP vaccine manufactured in Examples 6 and 7 were inoculated to the ICR mice in which 16 mice are grouped as the group 1. The blood was collected from the ICR mice after 28 days. Serum was separated from the blood. The antibody level against the tetanus antigen of the serum was measured using the EIA(Enzyme Immuno Assay) based on the unit of IU/ml and calculated as in geomean antibody titer. Thereafter, the titer of the combined vaccine was measured compared to the DTP vaccine.

Fig. 2c and 2d shows relative antibody titer of combined vaccine with repect to each antigen, and Table 1 shows the values of each titer.

### Test of antibody formation by Pertussis antigen

The combined vaccine and DTP vaccine manufactured in Examples 6 and 7 were inoculated to the ICR mice in which 16 mice are grouped as the group 1. The blood was collected from the ICR mice after 28 days. Serum was separated from the blood. The antibody level against pertussis antigen of the serum was measured using the EIA(Enzyme Immuno Assay) based on the unit of IU/ml and as in geomean antibody titer. Thereafter, the titer of the combined vaccine was measured compared to the DTP vaccine.

Fig. 2c and 2d shows relative antibody titer of combined vaccine with repect to each antigen, and Table 1 shows the values of each titer.

**Table 1**

| | DTwP vaccine (Aluminium Phosphate as an adsorbent) | DTwP vaccine (Aluminium Hydroxide as an adsorbent | DTwP-HepB combined vaccine (Sample A) | DTwP-HepB combined vaccine (Sample B) | Creterion of Titer |
|---|---|---|---|---|---|
| **Diphtheria** Potency(IU/ml) | >800 | 249.7 | 601.1 | 128.1 | Over |
| | | | | | 30 IU/ml |
| **Tetanus** Potency(IU/ml) | 176.5 | 196.0 | 216.9 | 230.7 | Over |
| | | | | | 40 IU/ml |
| **Pertussis** Potency(IPU/ml) | 20.2 | 3.2 | 25.4 | 5.5 | Over |
| | | | | | 8 IPU/ml |
| **Hepatitis B** Relative Potency(%) | - | - | 196.8 | 103.9 | Equal or over Value of Standard sample |

### Example 10.

### Comparative effectiveness test with monkey

A test was performed for proving the immunogenicity in a primate of the combined vaccine and comparing with the conventional single vaccine. The combined vaccine manufactured in Example 6 was vaccinated to six monkeys (group 2) formed of the same numbers of male and female monkeys, and each component vaccine was vaccinated to 6 monkeys(group 1) formed of the same numbers of male and female monkeys simultaneously. The same products as the products vaccinated to each group was re-vaccinated 30^{th} and 60^{th} days. The blood was collected from each experimental animal on 1^{st}, 29^{th}, 58^{th}, and 86^{th} days from the initial vaccination date, and the antibody titer against each disease was measured based on the ELISA method proper to the serum of the monkey.

Fig. 3 shows the geomean antibody titer against each antigen of each group.

### INDUSTRIAL APPLICABILITY

According to the present invention, it is directed to a method for manufacturing a combined vaccine for concurrently preventing the diseases such as diphtheria, tetanus, pertussis, and hepatitis B, etc. which should be prevented in an infant. By the present invention, it is possible to concurrently prevent the diseases such as diphtheria, tetanus, pertussis, and hepatitis B which should be prevented in an infant using the combined vaccine according to the present invention.

As the present invention may be embodied in several forms without departing from the spirit or essential characteristics thereof, it should also be understood that the above-described embodiments are not limited by any of the details of the foregoing description, unless otherwise specified, but rather should be construed broadly within its spirit and scope as defined in the appended claims, and therefore all changes and modifications that fall within the meets and bounds of the claims, or equivalences of such meets and bounds are therefore intended to be embraced by the appended claims.

## Claims

1. A method of manufacturing a combined vaccine comprising:
(a) providing individual protective antigens of diphtheria antigen, tetanus antigen, pertussis antigen and hepatitis B antigen,
(b) independently adsorbing each protective antigen to an adsorbent of aluminum hydroxide gel, and
(c) combining each independently adsorbed protective antigen

2. The method of claim 1, further comprising a step of:
adding additional aluminum hydroxide gel after combining each independently adsorbed protective antigen.

3. The method of claim 1 or 2, wherein the final aluminum ion concentration of the aluminum hydroxide gel is in the range of 0.5-1.25 mg/ml.

4. The method of claim 1, wherein the combined hepatitis B antigen is a recombinant hepatitis B surface antigen in an amount of 5-10 µg per dose.

5. The method of claim 4, further comprising a step of:
adding a neutral surfactant selected from the group consisting of polysorbate 20, polysorbate 80 and Triton^{®}X-100.

6. The method of claim 4, wherein said hepatitis B antigen is combined by stirring with an aluminum hydroxide at 2-8 °C for 3-20 hours.

7. The method of claim 1, wherein the diphtheria antigen is a diphtheria toxoid in an amount of 10-25 Lf per dose.

8. The method of claim 1, wherein the tetanus antigen is a tetanus toxoid in an amount of 1-5 Lf per dose.

9. The method of claim 1, wherein the pertussis antigen is
a purified pertussis antigen in an amount below 20 µgPN per dose; or
a whole cell pertussis antigen in an amount below 20 OU per dose.

10. The method of claim 9, wherein the purified pertussis antigen is pertussis toxoid, pertussis FHA (filamentous hemagglutinin) or a mixture thereof.

11. A combined vaccine as produced according to any of the methods claimed in claims 1 to 10.

## Patentansprüche

1. Verfahren zum Herstellen einer kombinierten Vakzine, umfassend:
(a) Bereitstellen individueller protektiver Antigene von Diphtherie-Antigen, Tetanus-Antigen, Pertussis-Antigen und Hepatitis-B-Antigen,
(b) unabhängiges Adsorbieren jedes protektiven Antigens auf ein Adsorptionsmittel aus Aluminiumhydroxidgel und
(c) Kombinieren jedes unabhängig adsorbierten protektiven Antigens.

2. Verfahren nach Anspruch 1, weiterhin umfassend einen Schritt:
Zugeben von zusätzlichem Aluminiumhydroxidgel nach dem Kombinieren jedes unabhängig adsorbierten protektiven Antigens.

3. Verfahren nach Anspruch 1 oder 2, wobei die Aluminiumionenendkonzentration des Aluminiumhydroxidgels in dem Bereich von 0,5-1,25 mg/ml liegt.

4. Verfahren nach Anspruch 1, wobei das kombinierte Hepatitis-B-Antigen ein rekombinantes Hepatitis-B-Oberflächenantigen in einer Menge von 5-10 µg pro Dosis ist.

5. Verfahren nach Anspruch 4, weiterhin umfassend einen Schritt:
Zugeben eines neutralen oberflächenaktiven Mittels, ausgewählt aus der Gruppe, bestehend aus Polysorbat 20, Polysorbat 80 und Triton^{®} X-100.

6. Verfahren nach Anspruch 4, wobei das Hepatitis-B-Antigen durch Rühren mit einem Aluminiumhydroxid bei 2-8°C für 3-20 Stunden kombiniert wird.

7. Verfahren nach Anspruch 1, wobei das Diphtherie-Antigen ein Diphtherietoxoid in einer Menge von 10-25 Lf pro Dosis ist.

8. Verfahren nach Anspruch 1, wobei das Tetanus-Antigen ein Tetanustoxoid in einer Menge von 1-5 Lf pro Dosis ist.

9. Verfahren nach Anspruch 1, wobei das Pertussis-Antigen ein
gereinigtes Pertussis-Antigen in einer Menge unterhalb von 20 µgPN pro Dosis oder
ein Ganzzell-Pertussis-Antigen in einer Menge unterhalb von 20 OU pro Dosis ist.

10. Verfahren nach Anspruch 9, wobei das gereinigte Pertussis-Antigen Pertussistoxoid, Pertussis-FHA (filamentöses Hämagglutinin) oder ein Gemisch davon ist.

11. Kombinierte Vakzine, wie gemäß einem der in den Ansprüchen 1 bis 10 beanspruchten Verfahren hergestellt.

## Revendications

1. Procédé de fabrication d'un vaccin combiné comprenant :
(a) fournir des antigènes protecteurs individuels de l'antigène de la diphtérie, de l'antigène du tétanos, de l'antigène de la coqueluche et de l'antigène de l'hépatite B,
(b) adsorber indépendamment chacun des antigènes protecteurs sur un adsorbant en gel d'hydroxyde d'aluminium, et
(c) combiner chacun des antigènes protecteurs adsorbés indépendamment.

2. Procédé selon la revendication 1, comprenant en outre une étape de :
addition du gel d'hydroxyde d'aluminium additionnel après avoir combiné chacun des antigènes protecteurs adsorbés indépendamment.

3. Procédé selon la revendication 1 ou 2, dans lequel la concentration finale en ions aluminium du gel d'hydroxyde d'aluminium est dans la plage de 0,5 à 1,25 mg/ml.

4. Procédé selon la revendication 1, dans lequel l'antigène de l'hépatite B combiné est un antigène de surface de l'hépatite B recombiné en quantité de 5 à 10 µg par dose.

5. Procédé selon la revendication 4, comprenant en outre une étape de :
addition d'un tensioactif neutre choisi dans le groupe constitué du polysorbate 20, du polysorbate 80 et du Triton® X-100.

6. Procédé selon la revendication 4, dans lequel ledit antigène de l'hépatite B est combiné en agitant avec de l'hydroxyde d'aluminium à 2 à 8 °C pendant 3 à 20 heures.

7. Procédé selon la revendication 1, dans lequel l'antigène de la diphtérie est une anatoxine diphtérique en quantité de 10 à 25 Lf par dose.

8. Procédé selon la revendication 1, dans lequel l'antigène du tétanos est une anatoxine tétanique en quantité de 1 à 5 Lf par dose.

9. Procédé selon la revendication 1, dans lequel l'antigène de la coqueluche est
un antigène purifié de la coqueluche en quantité inférieure à 20 µgPN par dose ; ou
un antigène de la coqueluche de type cellule entière en quantité inférieure à 20 OU par dose.

10. Procédé selon la revendication 9, dans lequel l'antigène purifié de la coqueluche est de l'anatoxine pertussique, de la FHA (hémagglutinine filamenteuse) de la coqueluche ou un mélange de celles-ci.

11. Vaccin combiné tel que produit selon l'un quelconque des procédés décrits dans les revendications 1 à 10.
